Europäisches Patentamt

European Patent Office

Office européen des brevets

(11) Numéro de publication: **0 005 392**
**B1**

(12) **FASCICULE DE BREVET EUROPEEN**

(45) Date de publication du fascicule du brevet: **02.06.82**

(51) Int. Cl.³: **A 61 N 1/36,** A 61 N 1/08

(21) Numéro de dépôt: **79400259.2**

(22) Date de dépôt: **24.04.79**

(54) Procédé de réglage d'un stimulateur cardiaque implantable et stimulateur pour la mise en oeuvre du procédé.

(30) Priorité: **05.05.78 FR 7813332**

(43) Date de publication de la demande:
**14.11.79 Bulletin 79/23**

(45) Mention de la délivrance du brevet:
**02.06.82 Bulletin 82/22**

(84) Etats contractants désignés:
**BE CH DE GB IT LU NL SE**

(56) Documents cités:
**DE - A - 2 216 043**
**FR - A - 2 135 774**
**FR - A - 2 167 560**
**FR - A - 2 342 722**
**FR - A - 2 366 012**
**FR - A - 2 374 024**

(73) Titulaire: **CARDIOFRANCE - COMPAGNIE FRANCAISE D'ELECTROCARDIOLOGIE**
**73, route de Neuilly**
**F-93160 Noisy Le Grand (FR)**

(72) Inventeur: **Buffet, Jacques**
**28, avenue Thiers**
**F-93340 Le Raincy (FR)**

(74) Mandataire: **Derambure, Christian**
**BUGNION ASSOCIES SARL 116, boulevard Haussmann**
**F-75008 Paris (FR)**

Courier Press, Leamington Spa, England.

# Procédé de réglage d'un stimulateur cardiaque implantable et stimulateur pour la mise en oeuvre du procédé

L'invention a pour objet un procédé de réglage d'un stimulateur cardiaque implantable, par voie extra-corporelle et un stimulateur cardiaque permettant la mise en oeuvre du procédé.

On connaît déjà des procédés de réglage de stimulateur cardiaque implantable et réglable, par voie extra-corporelle. Un tel stimulateur cardiaque comprend une sonde reliée électriquement à un boîtier dans lequel sont logés une source d'alimentation en énergie électrique: un amplificateur et un filtre; un module logique dont la fonction est l'élaboration des impulsions de stimulation, un étage de sortie dont la fonction est de délivrer sur la sonde des impulsions calibrées; et, enfin interrupteur à lame souple, normalement en position de circuit ouvert et court-circuité sous l'influence d'un champ magnétique, notamment d'origine extra-corporelle. La fonction de l'interrupteur magnétique est de commander le programme de fonctionnement du module logique, notamment un programme général dans lequel le stimulateur cardiaque fonctionne de façon classique à la demande et un programme particulier.

Dans un premier procédé connu de réglage du stimulateur cardiaque implantable, on approche du site où est implanté le stimulateur un aimant permanent ce qui a pour effet de court-circuiter l'interrupteur magnétique en provoquant donc la mise en route d'un programme logique particulier décrivant successivement les différents états de la caractéristique réglable à programmer. Au moment où la valeur particulière souhaitée est atteinte, on retire l'aimant de manière que l'interrupteur magnétique occupe la position ouverte et que le stimulateur cardiaque fonctionne normalement à la demande avec la caractéristique à la valeur programmée. Ce procédé a l'avantage de ne nécessiter qu'un appareil extra-corporel des plus réduits (aimant permanent). Cependant, il présente l'inconvénient d'être d'une mise en oeuvre mal commode puisque l'aimant doit être retiré rapidement au moment opportun. De plus, ce procédé ne permet de programmer qu'une caractéristique du stimulateur.

On a décrit dans le brevet français n° 2 342 722, un stimulateur cardiaque implantable dont les paramètres de fonctionnement peuvent être choisis de l'extérieur. Le stimulateur comporte un dispositif extérieur d'instruction conçu pour transmettre un train d'impulsions de sélection de paramètre. Il comporte en outre un moyen pour recevoir le train d'impulsions et pour produire un signal numérique en réponse au train. Le stimulateur comprend en outre un moyen de commande de mode permettant de modifier le mode de stimulation et transformer le stimulateur synchrone (à rythme variable) en stimulateur asynchrone (à rythme fixe). Ce moyen de commande de mode peut être actionné par une commande externe momentanée, par exemple un électro-aimant ou un composant semblable, de façon à provoquer le fonctionnement du stimulateur en mode à rythme fixe pour effectuer des essais. Ainsi, on commande par voie extra-corporelle le stimulateur pout qu'il fonctionne à une fréquence indépendante du rythme cardiaque autonome et on commande par voie extra-corporelle, l'émission ou, au contraire, la non émission continue d'un signal correspondant respectivement à une information binaire (1) ou (O) reçue et décelée en tant que telle par le stimulateur.

Cependant, un tel procédé de réglage d'un stimulateur et un tel stimulateur présentent des inconvénients.

En effet, les trains d'impulsions émis par le dispositif d'instruction, plus particulièrement un programmateur sont envoyés au stimulateur à une fréquence prédéterminée. Cette fréquence n'est donc pas fonction de la période de stimulation. Le programmeteur ne peut donc contrôler l'exactitude de la prise en compte de l'information par le stimulateur.

Pour pallier ces inconvénients, l'invention est telle que l'on décèle par voie extra-corporelle les impulsions successives émises par le stimulateur et que l'on commande l'émission ou la non émission continue dudit signal à partir de chaque impulsion décelée, et pendant la totalité de la période de stimulation.

Ainsi, on peut émettre ou non des signaux pendant le période de stimulation. Le train d'impulsions est synchronisé sur l'impulsion du stimulateur implanté et le stimulateur n'a donc pas à décider si l'onde détectée provient d'une activité cardiaque autonome ou provient du programmateur.

Dans le procédé proposé on assemble ces informations 1,0 en mots binaires et on réalise une clé de programmation par une partie de ce mot au moins une dénomination d'un paramètre du stimulateur à modifier par une autre partie de ce mot et la valeur modifiée du paramètre par une autre partie de ce mot.

Selon une caractéristique d'une forme de mise en oeuvre préférée du procédé de cette invention, elle est caractérisé par le fait que le signal émis par voie extra-corporelle est un train de N impulsions s'étendant entre deux impulsions de stimulation successives émises par le stimulateur et dont la période est inférieure à la période réfractaire du stimulateur.

Il est évident qu'ainsi on obtient un procédé très souple et sûr, d'une application beaucoup plus large, se rapportant à un nombre de paramètres et de valeur de réglage très importants.

L'invention propose également un stimulateur cardiaque, implantable et réglable par voie extra-corporelle pourvu d'un programmateur, pour la mise en oeuvre du procédé mentionné.

Les autres caractéristiques de l'invention résulteront de la description qui suivra en référence aux dessins annexés dans lesquels:

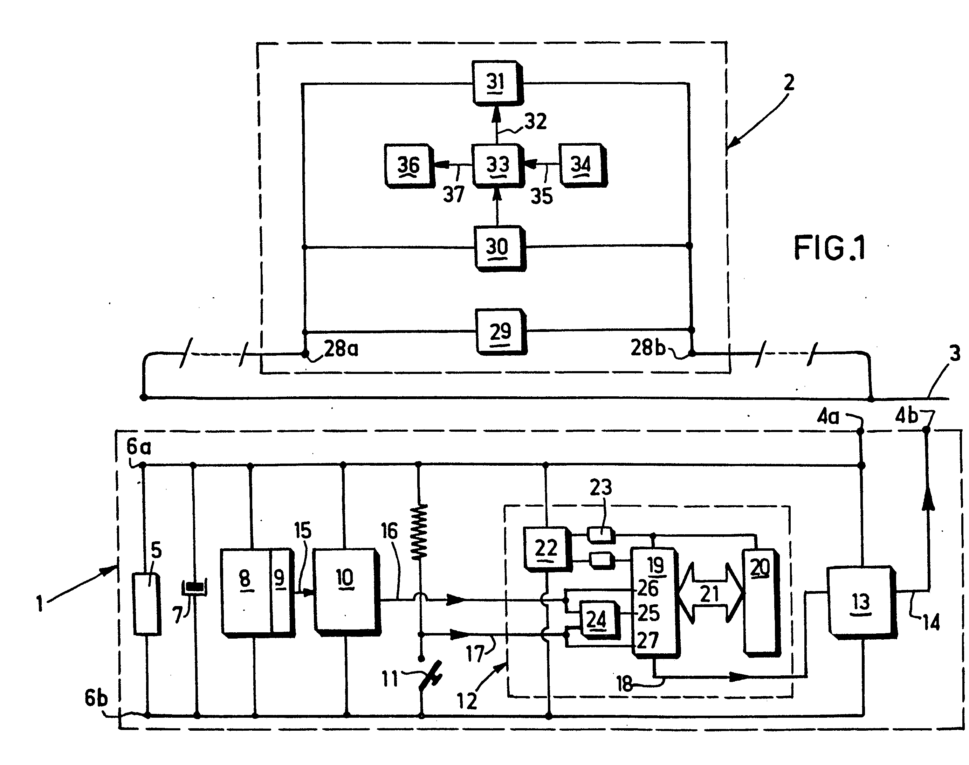

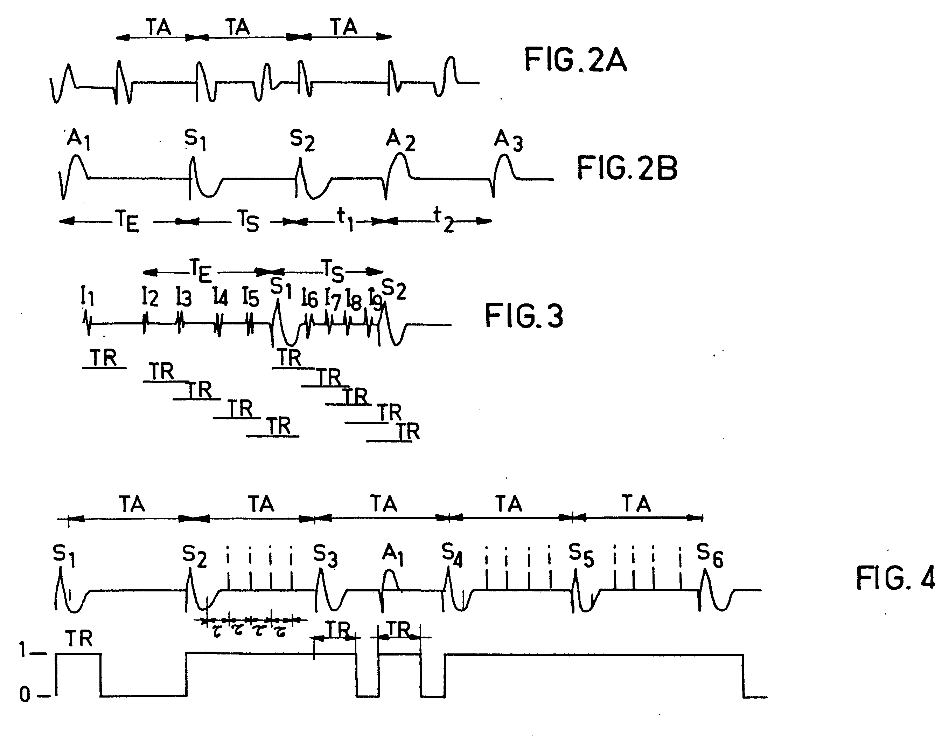

La figure 1 est une vue schématique illustrant l'ensemble fonctionnel stimulateur cardiaque implantable et le programmateur extra-corporel; les figures 2A et 2B sont des schémas illustrant le fonctionnement du stimulateur respectivement relais fermé et ouvert; la figure 3 est un schéma illustrant la période réfractaire du stimulateur; la figure 4 est un schéma illustrant le fonctionnement de l'ensemble programmateur-stimulateur suivant l'invention.

Le procédé de l'invention s'applique au réglage d'un stimulateur 1 implanté dans le corps d'un malade, par voie extra-corporelle. Selon ce procédé on commande par voie extra-corporelle le stimulateur pour qu'il fonctionne à une fréquence fixe indépendante du rythme cardiaque autonome; on décèle, par voie extra-corporelle, les impulsions successives émises par le stimulateur; par voie extra-corporelle, à partir de chaque impulsion décélée, et pendant la totalité de la période de stimulation, on commande l'émission d'un signal correspondant respectivement à une information binaire 1 ou au contraire, la non émission continue d'un tel signal correspondant à une information binaire 0. Ces informations sont reçues et décelées en tant que telles par le stimulateur.

Suivant l'invention, il est donc proposé un ensemble fonctionnel comportant en combinaison un stimulateur cardiaque 1, implantable, multiréglable et un programmateur 2, extra-corporel, placés au voisinage de la peau 3, du patient dans la zone d'implantation du stimulateur 1.

Le stimulateur 1 est du type connu sous le nom de stimulateur "sentinelle", destiné à être associé à une sonde cardiaque, non représentée, par deux électrodes 4a, 4b. Il comprend un boîtier, non représenté, et dans celui-ci une source d'énergie autonome 5, telle qu'une pile pourvue de connexions 6a et 6b dont l'une, soit 6a, par exemple, est reliée à l'électrode 4a. Entre les connexions 6a, 6b, sont placés en parallèle les éléments suivants: un condensateur 7; un amplificateur 8 et son filtre 9, un détecteur de niveau 10; un relais magnétique 11; un module logique désigné dans son ensemble par 12; et un étage de sortie 13 relié par une connexion 14 à l'électrode 4b. Des connexions 15, 16, 17, 18 relient respectivement l'amplificateur 8 et son filtre 9 au détecteur de niveau 10; ce dernier au module logique 12, le relais magnétique 11 également au module logique 12 et celui-ci à l'étage de sortie 13.

L'amplificateur 8 et son filtre 9 ont comme fonction d'amplifier et de filtrer les signaux qui sont détectés sur les électrodes 4a, 4b. Le coefficient d'amplification est notamment de l'ordre de 500 en tension environ. Le filtre 9 est notamment du type passe-bande centré sur une fréquence égale ou voisine de 60 hertz.

Le detecteur de niveau 10 apère une commutation lorsque le niveau en sortie de l'amplificateur 8 et du filtre 9 dépasse une valeur préfixée par exemple de l'ordre de 0.5 volt environ. Il résulte de ce dispositif que tout signal détecté sur les électrodes, accordé à la bande passante et dont l'amplitude une fois amplifiée dépassera le seuil de commutation du détecteur 10, entraîne la commutation de celui-ci.

Le module logique 12 a comme fonction d'élaborer des impulsions de stimulation en synchronisme avec les signaux de sortie du détecteur de niveau 10. Ce module logique comprend une unité centrale 19, une mémoire 20 à registre reliée à l'unité centrale 19, par un circuit "Bus" 21; une horloge 22 reliée aux connexions 6a, 6b; un circuit doubleur de tension 23 placé entre l'horloge 22 et l'unité centrale 19 d'une part et la mémoire 20, d'autre part; une porte logique "OU" 24, reliée à l'entrée interruption 25 de l'unité centrale 19 et aux connexions 16 et 17. La porte 24 est également reliée aux deux entrées "indicateurs" 26, 27 de l'unité centrale 19 sur laquelle vient la connexion 18. L'unité centrale 19 réalise donc un programme dont les instructions sont inscrites en langage hexadécimal dans la mémoire 20, via le circuit "Bus" 21. Le déroulement de ce programme est commandé par l'application des impulsions de l'horloge 22. préférentiellement, on utilise pour l'unité centrale des microprocesseurs.

L'étage de sortie 13 a comme fonction de déliverer aux électrodes 4a, 4b, des impulsions calibrées mises en forme selon les signaux en sortie du module logique 12.

Le relais magnétique 11 se présente notamment sous la forme d'un interrupteur à lame souple. Cet interrupteur est normalement en position de circuit ouvert tout en ètant susceptible d'être basculé en position de circuit fermé par des moyens de commande amovibles extra-corporels, notamment des moyens magnétiques et, en particulier, un organe produisant un champ magnétique tel qu'un aimant permanent.

Le programmateur 2 extra-corporel et amovible est destiné à être associé à des électrodes, non représentées, branchés sur des bornes 28a, 28b. Il comprend un boîtier, non représenté, dans lequel sont logés en parallèle entre les bornes 28a et 28b, une source d'énergie électrique autonome 29 telle qu'une pile; un amplificateur d'entrée 30 et un amplificateur de sortie 31. L'amplificateur d'entrée 30 est relié à l'amplificateur de sortie 31 par une connexion 32 sur laquelle est interposé un module logique 33. Un clavier de commande 34 est relié au module logique 33 par une connexion 35 et le module logique 33 est relié à un dispositif d'affichage alpha-numérique 36 par une connexion 37.

L'amplificateur d'entrée 30, pourvu d'un filtre, a comme fonction de recueillir les signaux provenant des électrodes via les bornes

28*a*, 28*b*. La bande passante de l'amplificateur est telle que seuls les signaux correspondant à une impulsion émise par le stimulateur 1 sont recueillis.

Le module logique 33 a comme fonction d'élaborer en réponse à l'amplificateur d'entrée 30, des "impulsions de commande" selon des instructions particulières données au moyen du clavier 34 et affichées sur le dispositif 36. Le clavier 34 permet donc de programmer manuellement le réglage souhaité de la variable du stimulateur 1.

L'amplificateur de sortie 31 a comme fonction de déliverer aux bornes 28*a*, 28*b* des impulsions calibrées et mises en forme selon les signaux en sortie du module logique 33.

Le stimulateur 1 fonctionne de la manière suivante:

Lorsque le relais magnétique 11 est court-circuité sous l'influence d'un champ magnétique extérieur, compte tenu du branchement de l'interrupteur à l'entrée "Interruption" 26 de l'unité centrale 19, il y a mise en route d'un programme particulier sans lequel le stimulateur émet des impulsions à une période constante TA indépendamment de toute activité cardiaque autonome et indépendante de la période de stimulation normale TS. La variation de la période TA dans le temps est représentative de l'état d'épuisement de la source d'énergie électrique 5 (la période augmente lorsque la source a'épuise). Ce mode de fonctionnement est illustré sur la figure 2A. Dans ce programme particulier, la sortie du détecteur de niveau 10, reliée par la connexion 16 à l'entrée "Indicateur" 26, est régulièrement explorée. Il en résulte que le module logique 12 est ainsi en mesure de déterminer si pendant la période TA, entre deux impulsions successives, existent ou n'existent pas des signaux tels que des trains d'interférences successives.

Sur la figure 2B est illustré le mode de fonctionnement du stimulateur 1 lorsque le relais magnétique 11 est ouvert c'est-à-dire qu'il n'est pas sous l'influence d'un champ magnétique extra-corporel. Dans ce cas, le stimulateur 1 remplit sa fonction normale thérapeutique de stimulation.

La figure 2B illustre un cas particulier; en A1, est représentée und onde cardiaque autonome. Si aucune activité cardiaque autonome ne fait suite à cette onde A1, le stimulateur émet, à l'issue d'une période dite période d'échappement TE, une impulsion de stimulation S1. Si aucune onde autonome n'est détectée après l'impulsion de stimulation S1, le stimulateur émet après la période TS une impulsion de stimulation S2. Une onde autonome A2 apparaissant après l'impulsion de stimulation S2, après un intervalle de temps t1 inférieur à TS, il y a recyclage du stimulateur 1 qui n'a pas à déliverer d'impulsion de stimulation. Si, après l'onde autonome A2, apparaît après un intervalle de temps t2, inférieure à TE, une autre onde autonome A3, il y a de même recyclage du stimulateur.

A ces modalités généralesviennent se superposer les modalités particulières résultant de la période réfractaire TR qui est l'intervalle de temps pendant lequel les signaux détectés ne sont pas pris en compte volontairement. La période réfractaire permet que les parasites électromagnétiques dont la période de répétition est inférieure à la période réfractaire TR ne provoquent pas le recyclage du stimulateur. Soit, par exemple, le signal I1 qui est une interférence électrique d'origine quelconque détectée pa le stimulateur. Ce signal n'est pas différencié par le stimulateur d'une onde cardiaque autonome et provoque donc le recyclage du stimulateur pour une période égale à la période d'échappement TE. A la suite du signal I1, le stimulateur respecte le période réfractaire TR. Si le signal I2 survient après un intervalle de temps supérieur à TR mais inférieur à TE, il provoque le recyclage du stimulateur pour la période d'échappement TE. Le signal I3 survient après un intervalle de temps inférieur à TR. Il ne s'ensuit aucun recyclage du stimulateur mais la période réfractaire est "reconduite" à partir de I3. De même, si le signal I4 survient dans la période réfractaire consécutive à I3, le phènomène déjà décrit en I3 se renouvelle à partir de I4. Il en est de même le signal I5. Après la période d'échappement TE à compter du signal I2, survient une impulsion de stimulation S1. Celle-ci déclenche à nouveau une période réfractaire TR. Le signal I6, dans cette période réfractaire, provoque également et comme vu précédemment la "reconduction" de celle-ci; il en est de même des signaux I7, I8 et I9. Après une période de stimulation TS à compter de l'impulsion de stimulation S1, le stimulateur émet une impulsion de stimulation S2.

Le programmateur 2 fonctionne, en vue du réglage d'une variable du stimulateur, en combinaison avec ce dernier. Dans ce cas, une impulsion de stimulation émise par le stimulateur cardiaque 1 est décelée par les électrodes associées au programmateur 2 et reconnue comme telle par l'amplificateur d'entrée 30. Les indications d'une variable à régler d'une part, et d'une valeur à donner à cette variable d'autre part, appliquées au module logique 33 par l'intermédiaire du clavier 34, permettent qu'à chaque impulsion adressée par l'amplificateur d'entrée 30 au module logique 33 corresponde un signal ou une absence de signal pendant la durée séparant deux impulsions successives. Ce signal ou cette absence de signal est adresseé à l'amplificateur de sortie 31 qui les applique aux électrodes associées au programmateur pour être ensuite reconnu comme tel par le stimulateur cardiaque implanté 1.

En pratique, la programmation ou le réglage est rendue possible lorsque le relais magnétique 11 est court-circuité et que le stimulateur 1 émet des impulsions à la période TA. Etant donné, comme on le verra ultérieurement, que le programmateur peut éventuellement fonctionner lorsque le stimulateur fonctionne selon le programme général, on préfère que l'aimant permanent créant le champ magnétique en vue de court circuiter le relais magnétique 11 soit matériellement distinct du programmateur. On conçoit cependant que cet aimant permanent puisse constituer un ensemble monobloc avec le programmateur proprement dit.

Les électrodes associées au programmateur sont placées de manière connue permettant de recueillir les électro-cardiogrammes. Plus précisément, les électrodes sont disposées au contact de la peau du patient en deux points suffisamment éloignés l'un de l'autre, notamment la bras droit et le bras gauche. L'amplificateur d'entrée 30 distingue parmi les signaux d'entrée les impulsions de stimulation des ondes autonomes et des autres signaux du fait de sa bande passante calculée à cet effet.

Le signal de sortie émis par l'amplificateur 31 est, suivant une caractéristique préférée d'un mode de réalisation de l'invention, un train de N impulsions d'une période $\tau$ inférieure à la période réfractive. Il en résulte que, selon la présence ou l'absence de signal émis par l'amplificateur de sortie 31 c'est-à-dire la présence ou l'absence du train de N impulsions, le stimulateur 1 voit ou, au contraire ne voit pas sa période rèfractaire reconduite sur toute la période TA séparant deux impulsions successives. Le relais magnétique 11 ayant été court-circuité, la sortie du détecteur de niveau 10 où apparaît la présence ou l'absence du train d'impulsions, est explorée par le module logique 12 qui traduit comme étant une information 1 ou 0, la reconduction ou non de la période réfractaire entre deux impulsions successives.

A cet effet, dès la première transmission de l'information 1, le module logique 12 se placera en mode programmation et pourra alors enregistrer les informations successives constituant un mot binaire auquel correspond un réglage particulier du stimulateur.

Sur la figure 4 est illustrée, à titre d'exemple la transmission du mot binaire 1 0 1 1. S1 est une impulsion de stimulation. A partir de S1 et pendant la durée TR court la période réfractaire qui cesse jusqu'à l'émission d'une impulsion de stimulation S2 à un temps TA après S1. La transmission commence après S2. Le programmateur envoie en réponse à l'impulsion de stimulation S2, un train de cinq impulsions qui couvre la période TA entre l'impulsion de stimulation S2 et l'impulsion de stimulation S3. Il s'ensuit, compte tenu de la période $\tau$ de ces impulsions $i$, que la période réfractaire du stimulateur est reconduite en totalité entre les impulsions de stimulation S2 et S3. Après l'impulsion de stimulation S3, la période réfractaire se prolonge pendant la durée TR. Etant donné qu'à partir de l'impulsion de stimulation S3, le programmateur n'émet aucun train d'impulsions, la période réfractaire n'est pas reconduite et tombe donc à un niveau 0. Si une onde autonome A1 apparaît dans la période TA à compter et après l'impulsion S3, il s'ensuit que le stimulateur retrouve pour TR sa période réfractaire qui cesse ensuite jusqu'à ce que soit émise l'impulsion de stimulation S4 au bout d'une durée TA après l'impulsion de stimulation S3. Il est donc clair qu'entre les deux impulsions de stimulation S3 et S4 il n'y a pas eu reconduction continue et totale de la période réfractaire. On notera que les différentes périodes respectivement TA et TR sont telles que même l'apparition d'une onde autonome ne permet aucun doute possible quant à la discontinuité de la reconduction de la période réfractaire.

A partir de l'impulsion de stimulation S4, le programmateur émet un train de N impulsions et ce, jusqu'à ce que soit émise après la durée TA une impulsion de stimulation S5. La période réfractaire est donc continuellement reconduite entre S4 et S5. De même entre S5 et S6.

Bien entendu, le code de transmission des informations peut faire l'objet de nombreuses variantes. On peut cependant retenir comme exemple un mot binaire à trois parties. La première est une clé de programmation, la deuxième est la dénomination du paramètre à programmer parmi les différents paramètres programmables et la troisième est la nouvelle valeur donnée au paramètre.

Le cas échéant, on conçoit l'existence de moyens de contrôle pour éviter les erreurs de programmation.

Il est clair que les possibilités de programmation sont extremement variées. Il n'est donc pas possible d'en donner une liste limitative. A titre d'exemples cependant, il est possible de programmer les caractéristiques suivantes:
— période de stimulation en seize échelons entre 500 et 1300 millisecondes.
— largeur de l'impulsion de stimulation en seize échelons de 0,1 à 1,6 millisecondes.
— amplitude de l'impulsion de stimulation en trois échelons de 2,5—5 et 7,5 volts.
— sensibilité de l'amplificateur du stimulateur en quatre échelons de 0,5—1,5—4 et 10 millivolts.
— période réfractaire en huit échelons de 50 à 450 millisecondes.
— période d'échappement en seize échelons de 500 à 1300 millisecondes.

Chacune des caractéristiques correspond donc à une valeur binaire — un mot de quatre digits binaires — qui est stocké dans un registre de données de l'unité centrale et utilisé par le programme général.

Outre une fonction d'émission d'information de commande, le programmateur est susceptible d'avoir une fonction de réception d'information relative au fonctionnement du stimulateur. Dans ce cas, le programmateur fonctionne avec ou sans le relais magnétique 11 court-circuité c'est-à-dire avec ou sans champ magnétique et ce, en fonction des besoins. Il en résulte la possibilité de recevoir sur le dispositif d'affichage 36, les informations suivantes:

— Rythme de stimulation du stimulateur ce qui permet d'évaluer le degré d'épuisement de la source énergétique 5.

— Mesure de la largeur de l'impulsion de stimulation émise par le stimulateur.

— Mesure de l'amplitude de l'impulsion de stimulation émise par le stimulateur.

Enfin, le programmateur est susceptible d'émettre une impulsion synchronisée avec un retard variable et réglable sur l'impulsion de stimulation du stimulateur et ce, pour différents usages souhaités.

**Revendications**

1. Procédé de réglage d'un stimulateur cardiaque implantable et réglable par voie extra-corporelle dans lequel on commande par voie extra-corporelle le stimulateur pour qu'il fonctionne à une fréquence fixe indépendante du rythme cardiaque autonome et on commande par voie extra-corporelle l'émission ou, au contraire, la non émission continue d'un signal correspondant respectivement à une information binaire (1) ou (0) reçue décelée en tant que telle par le stimulateur, caractérisé par le fait qu'on décèle par voie extra-corporelle les impulsions successives émises par le stimulateur et que l'on commande l'émission ou la non émission continue dudit signal à partir de chaque impulsion décelée, et pendant la totalité de la période de stimulation.

2. Procédé selon la revendication 1, caractérisé par le fait que le signal émis par voie extra-corporelle est un train de N impulsions s'étendant entre deux impulsions de stimulation successives émises par le stimulateur et dont la période est inférieure à la période réfractaire du stimulateur.

3. Stimulateur pour la mise en oeuvre du procédé selon la revendication 1, comprenant une source d'énergie électrique autonome (5), un amplificateur (8) et un filtre (9), un détecteur de niveau (10), un étage de sortie (13), caractérisé par le fait qu'il comporte entre le détecteur de niveau (10) et l'étage de sortie (13), un module logique (12) relié à un relais magnétique (11), tel que le module logique comporte une unité centrale (19) ayant un registre de données (20) dans lequel est stocké un certain nombre de caractéristiques différentes du stimulateur, chacune d'elles étant réglée à une valeur définie.

4. Stimulateur cardiaque selon la revendication 3, caractérisé par le fait que le détecteur de niveau est relié à l'entrée "Indicateur" de l'unité centrale de manière que lorsque le relais magnétique est court-circuité, la sortie du détecteur de niveau soit régulièrement explorée par le module logique.

5. Stimulateur pour la mise en oeuvre du procédé selon l'une quelconque des revendications 1 et 2, caractérisé par le fait qu'il est associé à un programmateur (2) destiné au réglage d'une des caractéristiques réglables du stimulateur cardiaque implantable, comprenant entre deux bornes (28a, 28b) auxquelles peuvent être associées des électrodes, une source d'énergie électrique autonome (29), un amplificateur d'entrée (30), un amplificateur de sortie (31); l'amplificateur d'entrée étant relié à l'amplificateur de sortie par une connexion ( 32), sur laquelle est interposé un module logique (33) auquel est connecté un clavier de commande (34).

6. Stimulateur selon la revendication 5, caractérisé par le fait que l'amplificateur d'entrée du programmateur associé a une bande passante telle qu'il ne retient que les seules impulsions de stimulation émises par le stimulateur cardiaque implanté et auquel est associé le programmateur.

7. Stimulateur selon l'une quelconque des revendications 5 et 6, caractérisé par le fait que le programmateur associé comporte en outre un dispositif d'affichage (36) associé au module logique (33) du programmateur.

8. Stimulateur selon l'une quelconque des revendications 5 à 7, caractérisé par le fait que le module logique (33) du programmateur a comme fonction d'elaborer et d'adresser à l'amplificateur de sortie un train d'impulsions ou l'absence d'un train d'impulsions de période inférieure à la période réfractaire du stimulateur et ce, à partir d'une impulsion de stimulation reçue par l'amplificateur d'entrée (30).

9. Stimulateur selon l'une quelconque des revendications 5 à 8, caractérisé par le fait que le programmateur associé fonctionne en réception de manière à permettre l'affichage sur le dispositif d'affichage de caractéristiques de fonctionnement du stimulateur.

10. Stimulateur selon l'une quelconque des revendications 5 à 9, caractérisé par le fait que le programmateur associé comporte en outre une source magnétique faisant partie intégrante ou non du programmateur.

**Patentansprüche**

1. Verfahren zum Einstellen eines implantierbaren und über einen außerkörperlichen Weg einstellbaren Herzschrittmachers, bei welchem der Schrittmacher, damit er bei einer festen, vom eigenständigen Herzrythmus unabhängigen Frequenz arbeitet, und das kontinuierliche Abgeben oder Nichtabgeben eines Signals über einen außerkörperlichen Weg gesteuert werden, welches Signal einer binären Information (1) oder (0) entspricht, die als solche vom Schrittmacher empfangen und erfasst wird, dadurch gekennzeichnet, daß über den außerkörperlichen Weg die aufeinanderfolgenden, vom Schrittmacher abgegebenen Impulse erfasst werden und daß kontinuierliche Abgeben oder Nichtabgeben dieses Signals von jedem erfassten Impuls an und während der gesamten Stimulierungsperiode gesteuert wird.

2. Verfahren nach Anspruch 1, dadurch gekennzeichnet, daß das über den außerkörperlichen Weg abgegebene Signal'ein Zug von N-Ipulsen ist, der sich zwischen zwei aufeinanderfolgenden, vom Schrittmacher abgegebenen Stimulierungsimpulsen erstreckt und dessen Periode geringer ist als die refraktäre Periode des Schrittmachers.

3. Herzschrittmacher zur Ausführung des Verfahrens nach Anspruch 1, mit einer elektrischen eigenständigen Energiequelle (5), einem Verstärker (8) und einem Filter (9) einem Pegeldetektor (10), einer Ausgangsstufe (13), dadurch gekennzeichnet, daß er zwischen dem Pegeldetektor (10) und der Ausgangsstufe (13) eine Logik (12) aufweist, die mit einem Magnetrelais (11) verbunden ist und die eine Zentraleinheit (19) aufweist, welche ein Datenregister (20) besitzt, in welchem eine bestimmte Anzahl verschiedener Charakteristika des Schrittmachers gespeichert ist, von denen jedes Charakteristikum auf einen bestimmten Wert eingestellt ist.

4. Schrittmacher nach Anspruch 3, dadurch gekennzeichnet, daß der Pegeldetektor (10) mit dem Eingang "Anzeige" der Zentraleinheit (19) derart verbunden ist, daß, sobald das Magnetrelais (11) kurzgeschlossen ist, der Ausgang des Pegeldetektors von der Logik (12) regelmäßig abgefragt wird.

5. Herzschrittmacher zur Ausführung des Verfahrens nach einem der Ansprüche 1 oder 2, dadurch gekennzeichnet, daß er mit einer Programmiervorrichtung (2) verbunden ist, die zum Einstellen eines der einstellbaren Charakteristika des implantierbaren Herzschrittmachers bestimmt ist und die zwischen zwei Klemmen (28a, 28b) mit denen Elektroden verbunden sein können, eine elektrische, eigenständige Energiequelle (29), einen Eingangsverstärker (30) und einen Ausgangsverstärker (31) aufweist, wobei der Eingangsverstärker mit dem Ausgangsverstärker über eine Verbindung (32) verbunden ist, auf der ein Logikelement (33) zwischengeschaltet ist, mit welchem eine Steuertastatur (34) verbunden ist.

6. Schrittmacher nach Anspruch 5, dadurch gekennzeichnet, daß der Eingangsverstärker (30) der Programmiervorrichtung (2) einen Bandpass besitzt, der nur die einzelnen vom implantierten Herzschrittmachdr abgegebenen Stimulierungsimpulse nicht durchlässt und mit dem die Programmiervorrichtung verbunden ist.

7. Schrittmacher nach einem der Ansprüche 5 oder 6, dadurch gekennzeichnet, daß die Programmiervorrichtung (2) ferner eine Vorrichtung zur Datendarstellung (36) aufweist, die mit dem Logikelement (33) der Programmiervorrichtung verbunden ist.

8. Schrittmacher nach einem der Ansprüche 5 bis 7, dadurch gekennzeichnet, daß das Logikelement (33) der Programmiervorrichtung (2) zum Verarbeiten und zum Adressieren eines Ipulszuges oder des Nichtvorhandenseins eines Impulszuges mit einer Periode, die geringer ist als die refraktäre Periode des Schrittmachers, an den Ausgangsverstärker (31) dient, und zwar vom Eingangsverstärker (30) empfangenen Stimulierungsimpuls an.

9. Schrittmacher nach einem der Ansprüche 5 bis 8, dadurch gekennzeichnet, daß die Programmiervorrichtung (2) beim Empfang derart arbeitet, daß sie auf der Vorrichtung zur Datendarstellung (36) die Datendarstellung der Funktionscharakteristika des Schrittmachers ermöglicht.

10. Schrittmacher nach einem der Ansprüche 5 bis 9, dadurch gekennzeichnet, daß die Programmiervorrichtung (2) ferner eine Magnetquelle aufweist, die integraler oder nicht integraler Bestandteil der Programmiervorrichtung ist.

## Claims

1. Method for regulating a cardiac stimulator which can be implanted and regulated by extra-corporal means in which the stimulator is controlled by extra-corporal means in order that it operates at a fixed frequency independent of the autonomous cardiac rhythm and the emission or, on the contrary, the continuous non-emission of a signal corresponding respectively to binary information (1) or (0) received and detected as such by the stimulator is controlled by extra-corporal means, characterised by the fact that the successive pulses emitted by the stimulator are detected by extra-corporal means and that the emission of continuous non-emission of the said signal is controlled from each pulse detected and throughout the entire period of stimulation.

2. Method according to claim 1, characterised by the fact that the signal emitted by extra-corporal means is a train of N pulses extending between two successive stimulation pulses emitted by the stimulator and the period of which is less than the refractory period of the stimulator.

3. Stimulator for carrying out the method according to claim 1, comprising an autonomous source of electrical energy (5), an amplifier (8) and a filter (9), a level detector (10), an output stage (13), characterised by the fact that between the level detector (10) and the output stage (13) it comprises a logic module (12) connected to a magnetic relay (11), such that the logic module comprises a central unit (19) having a data register (20) in which a certain number of different characteristics of the stimulator is stored, each of the latter being regulated at a defined value.

4. Cardiac stimulator according to claim 3, characterised by the fact that the level detector is connected to the "indicator" input of the central unit so that when the magnetic relay is short-circuited, the output of the level detector is regularly explored by the logic module.

5. Stimulator for carrying out the method according to one of claims 1 and 2, characterised by the fact that it is associated with a programmer (2) intended for regulating one of the adjustable characteristics of the implantable cardiac stimulator, comprising between two terminals (28a, 28b), with which electrodes may be associated, an autonomous source of electrical energy (29), an input amplifier (30), an output amplifier (31); the input amplifier being connected to the output amplifier by a connection (32) in which is interposed a logic module (33) to which a control key (34) is connected.

6. Stimulator according to claim 5, characterised by the fat that the input amplifier of the associated programmer has a pass band such that it retains only the single stimulation pulses emitted by the implanted cardiac stimulator and with which the programmer is associated.

7. Stimulator according to one of claims 5 and 6, characterised by the fact that the associated programmer also comprises a display device (36) associated with the logic module (33) of the programmer.

8. Stimulator according to one of claims 5 to 7, characterised by the fact that the function of the logic module (33) of the programmer is to elaborate and address to the output amplifier a train of pulses or the absence of a train of pulses whereof the period is less than the refractory period of the stimulator and this is from a stimulation pulse received by the input amplifier (30).

9. Stimulator according to one of claims 5 to 8, characterised by the fact that the associated programmer operates for receiving in order to allow the display of operating characteristics of the stimulator on the display device.

10. Stimulator according to one of Claims 5 to 9, characterised by the fact that the associated programmer also comprises a magnetic source forming an integral or non-integral part of the programmer.

FIG.1

FIG.2A

FIG.2B

FIG.3

FIG.4